# EUROPEAN PATENT APPLICATION

(11) **EP 3 382 040 A2**
(43) Date of publication of application: **03.10.2018**
(21) Application number: 18159299.9
(22) Date of filing: 01.12.2014
(51) Int. Cl.: C12Q 1/689, C12Q 1/6883

(54) **GENETIC MARKERS FOR DIAGNOSIS OF TUBERCULOSIS CAUSED BY MYCOBACTERIUM TUBERCULOSIS**

(30) Priority: 03.12.2013 IN 5572CH2013
(62) Divisional of application: 14825438.6
(71) Applicant: Kusuma School of Biological Sciences, New Delhi, Delhi 110016 (IN); European Molecular Biology Laboratory, 69117 Heidelberg (DE)
(72) Inventor: RAHMAN, Syed Asad, Impington Cambridge CB24 9PX (GB); AHMAD, Javeed, 192302 Srinagar, Jammu and Kashmir (IN); EHTESHAM, Nasreen Z., 110016 New Delhi (IN); HASNAIN, Seyed E., 110016 New Delhi (IN)
(74) Representative: Zwicker, Jörk

(57) **Abstract**

The application is related to novel signature sequences for diagnosis of *Mycobacterium tuberculosis* in clinical samples with 100% specificity and a very high degree of sensitivity.

## Description

### Related Application

This application is related to and takes priority from the Indian Provisional Application 5572/CHE/2013 filed on December 3, 2013 and is incorporated herein in its entirety.

### Field of the Invention

The application is related to novel signature sequences for diagnosis of *Mycobacterium tuberculosis* in clinical samples. These signature sequences have the ability to differentiate *Mycobacterium tuberculosis* DNA from other mycobacterial species by PCR with 100% specificity and very high sensitivity.

### Background of the Invention

Tuberculosis (TB) is a major global health problem with an alarming rate of mortality associated with it. It is one of the leading infectious diseases caused by bacteria taking one human life every 15-20 seconds globally. Estimates of 2011 reveal that there are almost 9 million new cases and 1.4 million TB deaths (Global Tuberculosis Report 2012, WHO 2013). The disease is caused by *Mycobacterium tuberculosis,* a member of the genus Mycobacterium, while in a few cases *Mycobacterium bovis* has been reported to be the causal organism. More than 100 mycobacterium species are known and among them only a few are pathogenic for humans.

Conventional diagnostic methods include examination of sputum smear under a microscope for acid-fast mycobacteria and radiological reading of lungs. However, in most cases the sputum smear examination turns out to be negative for the bacteria due to early stages of infection and lung changes are not readily visible on an x-rays until several months into the infection.

Diagnosis of mycobacteria-related disease poses difficulties for several reasons which have been recognized by researchers and clinicians over the years. Firstly, these bacteria are in small numbers and are already at a contagious stage when they become detectable by conventional methods. Pulmonary disease caused by different mycobacteria are not readily detectable clinically or radiologically. Detection of organism in culture achieves 100% specificity but the growth of mycobacteria in culture takes about 3-6 weeks (Bates et al., Am. Respir.134, 415-417, 1986) thus making the process time-consuming. In addition, repeated cultures may be required to ensure success.

Several molecular tests for tuberculosis have been developed in the past. These include the Gen-probe 'Amplified mycobacterium direct test' by Abbe et al (J. Clin. Mincrobiol. 31, 3270, 1993), ligase chain reaction (LCR) (J. Clin. Microbiol. 35, 2424, 1997), PCR based Roche AmplicorTB test (J. Clin. Microbiol. 33, 1832, 1995) and IS6110 based detection (J. Clin. Microbiol. 28, 2668, 1990).

US5168039 discloses the IS6110 based detection of *M*. *tuberculosis* wherein a repetitive DNA segment specific for members of *M*. *tuberculosis* complex is used for the diagnosis. While IS6110 based detection system has been shown to have high level of specificity, there are also reports on false positive detections of 3 to 20% making it unreliable (J. Clin. Microbiol. 32, 277, 1994). In addition, lack of IS6110 sequence in some *M*. *tuberculosis* strains may also limit the use of the same for diagnostic tests routinely (Tuber. Lung Dis. 76, 550, 1995). US7638309 provides detection of mycobacteria in clinical specimens in the intergenic region between methyl mycolic acid synthase genes mmaA1 and mmaA2 and the flanking region in mmaA1 and mmaA2 genes.

Thus, it appears that there is a paucity of simple, rapid and reliable tests that can specifically detect *M*. *tuberculosis* during early stages of the disease. The present invention has identified 'signature sequences' that can differentiate *M*. *tuberculosis* from a large number of other mycobacterial DNA. These 'signature sequences' are used in detection of early disease in clinical samples of patients.

### Summary of the Invention

The invention provides novel signature sequences for diagnosis of Mycobacterium species (sps) in clinical samples with 100% specificity and a very high degree of sensitivity.

In one aspect, the invention provides a nucleotide sequence capable of selectively detecting pathogenic Mycobacterium sps using oligonucleotide primers corresponding to the signature sequence selected from SEQ ID NO: 1, 2, 3 or 4.

In another aspect the invention provides a method of detecting pathogenic mycobacterium sps in a clinical sample, said method comprising the steps of:
a. removal of contaminants from the clinical sample by conventional methods;
b. extraction of genomic DNA from the contaminant-free clinical sample;
c. designing a set of specific oligonucleotide primers capable of specifically detecting SEQ ID NO: 1, 2, 3 or 4 for use in RT-PCR;
d. analyzing PCR product by electrophoresis or specific probe nucleotide sequence complementary to SEQ ID NO: 1, 2, 3 or 4.

The set of oligonucleotide primers of the invention are selected from
(i) 5' ATGCAGGTTGCGACTGTACACCCGG 3' (SEQ ID NO: 5) 3' GGCCGCTCTTGTTCTTCGTCGGAT 5' (SEQ ID NO: 6)
(ii) 5' GTGTTTGCGTTGAGTAATAATCTGAACCGTGT 3' (SEQ ID NO: 7) 3' AGCCAATTCCAGCACGATGTCGCC 5' (SEQ ID NO: 8)
(iii) 5' ATGCAGGTTGCGACTGTACACCCGG 3' (SEQ ID NO: 9) 3' GGCCGCTCTTGTTCTTCGTCGGAT 5' (SEQ ID NO: 10)
(iv) 5' TGTACCGCGTGCCCGACGATTTG 3' (SEQ ID NO: 11) 3' ACAGGCAGCTAACAGGGCGTCGG 5' (SEQ ID NO: 12)
(v) a set of oligonucleotide primers complementary to (i), (ii), (iii) or (iv)
or
(vi) a set of oligonucleotide primers comprising of sequence containing any 10 consecutive bases from one of the sequences selected from SEQ ID NO: 1, 2, 3 or 4.

In yet another aspect, the invention provides a kit for the detection of pathogenic mycobacterium sps in clinical samples, said kit comprising set of oligonucleotide primers selected from
(i) 5' ATGCAGGTTGCGACTGTACACCCGG 3' (SEQ ID NO: 5) 3' GGCCGCTCTTGTTCTTCGTCGGAT 5' (SEQ ID NO: 6)
(ii) 5' GTGTTTGCGTTGAGTAATAATCTGAACCGTGT 3' (SEQ ID NO: 7) 3' AGCCAATTCCAGCACGATGTCGCC 5' (SEQ ID NO: 8)
(iii) 5' ATGCAGGTTGCGACTGTACACCCGG 3' (SEQ ID NO: 9) 3' GGCCGCTCTTGTTCTTCGTCGGAT 5' (SEQ ID NO: 10)
(iv) 5' TGTACCGCGTGCCCGACGATTTG 3' (SEQ ID NO: 11) 3' ACAGGCAGCTAACAGGGCGTCGG 5' (SEQ ID NO: 12)
(v) a set of oligonucleotide primers complementary to (i), (ii), (iii) or (iv)
or
(vi) a set of oligonucleotide primers comprising of sequence containing any 10 consecutive bases from one of the sequences selected from SEQ ID NO: 1, 2, 3 or 4.

Furthermore, the invention provides a method of detecting pathogenic mycobacterium sps in a clinical sample wherein the sample is isolated from individuals vaccinated against tuberculosis. It also provides a method of detecting pathogenic mycobacterium sps in a clinical sample wherein the sample is isolated from individuals treated against tuberculosis.

### Brief Description of Figures

Fig. 1A: Amplification of SS1 at low and varied DNA template concentrations.
Fig. 1B: Amplification of SS2 at low and varied DNA template concentrations.
Fig. 1C: Amplification of SS3 at low and varied DNA template concentrations.
Fig. 1D: Amplification of SS4 at low and varied DNA template concentrations.
Fig. 2: Amplification of signature sequences SS1, SS2, SS3 and SS4 from patient sputum samples.
Fig. 3: Amplification of signature sequences SS1 and SS3 from patient blood samples.

### Details of the Invention

The present invention relates to detection of pathogenic Mycobacterium species using signature sequences SEQ ID NO: 1, 2, 3 or 4 with a high degree of sensitivity and 100% specificity.

In one embodiment, the invention provides novel DNA diagnostic markers for specific detection of *Mycobacterium tuberculosis* which causes tuberculosis.

A three-pronged approach was carried out to identify novel DNA diagnostic marker for detection of pathogenic mycobacterium sps, especially, *Mycobacterium tuberculosis.* First step provides an *in-silico* approach to identify and shortlist potential sequences of Mycobacterium, unique and exclusive to pathogenic mycobacterium sps, especially *Mycobacterium tuberculosis.* The criteria used for selection of the potential sequences are presented below which involves comparative proteomic analysis of 13 mycobacterium species:
i. Strict pathogens (the most virulent pathogens) such as *Mycobacterium tuberculosis, Mycobacterium laprae, Mycobacterium ulcernus* and *Mycobacterium bovis.*
ii. Opportunistic pathogens, which belong to Non Tuberculous Mycobacteria (NTM) group, can cause pulmonary and other disseminated infections in immune compromised individuals (Infect. Genet. Evol. 12, 832, 2012; Appl. Environ. Microbiol. 79, 825, 2013). *Mycobacterium marinum, Mycobacterium avium, Mycobacterium intracellulare, Mycobacterium aviumpara tuberculosis and Mycobacterium abscessus,* cause opportunistic pulmonary infection in human whereas *Mycobacterium avium* subspecies *paratuberculosis* (MAP), the third member of MAC is the suspected causative agent of Crohn's disease in human (Appl. Environ. Microbiol. 79, 825, 2013; Crit. Rev. Microbiol. 38, 52, 2012).
iii. Non-pathogenic group includes *Mycobacterium gilvum, Mycobacterium vanbaalenii,Mycobacterium smegmatisand Mycobacterium indicus pranii,* which does not cause disseminated infections even in immune compromised individuals (BMC Microbiol. 10, 237, 2010; Infect. Genet. Evol., 12, 853, 2012; Br. J. Exp. Pathol. 52, 627, 1971).

The following bioinformatics process flow resulted in the identification of potential gene markers of the invention.
a) Perform all against all NCBI BLAST (J Mol Biol., 215, 403, 1990) on the protein sequences from the selected genomes.
b) Perform all against all NCBI BLAST ((J Mol Biol., 215, 403, 1990) on the nucleotide sequences from the selected genomes.
c) Identify Protein Domains from Pfam (Comp. Genomics, 396, 43, 2007) and GENE-3D/CATH (Nucleic Acids Research, 40, D465, 2012) using InterPro (Nucleic Acids Research, 40, D306, 2012).
d) Classify sequences into three categories namely i) CLASS 1 and ii) CLASS 2 as potential hits and iii) rest as non-hits.

The above process flow resulted in the classification of the potential hits into class 1 and class 2. As per the present invention, class 1 are genes unique to the organism of interest based on the fact that they do not share protein domain and protein sequence identity of more than 20% and nucleotide sequence identity of more than 35% with any other organism in the selected organism list. In another embodiment, class 2 genes are those that do not share protein domain and protein sequence identity of more than 20% and nucleotide sequence identity of more than 35% with any other organism in the selected organism list.

Table 1 provides potential candidate genes carrying 'signature sequences' based on the bioinformatics process flow.

**Table 1: Potential "signature sequences" carrying candidate genes**

| **H37Rv Gene Identifiers** | **Class** | **H37Rv protein description** |
|---|---|---|
| **Rv1507A** | 1 | Hypothetical protein |
| **Rv1509** | 1 | Hypothetical protein |
| **Rv2645** | 1 | Hypothetical protein |
| **Rv2653c** | 1 | Possible PhiRv2 prophage protein |
| **Rv2654c** | 1 | Possible PhiRv2 prophage protein |
| **Rv2658c** | 1 | Possible prophage protein |
| **Rv0064A** | 2 | Possible antitoxin VapB1 |
| **Rv0078B** | 2 | Hypothetical protein |
| **Rv0397A** | 2 | Hypothetical protein |
| **Rv0456B** | 2 | Possible antitoxin MazE1 |
| **Rv0959A** | 2 | Possible antitoxin VapB9 |
| **Rv1366A** | 2 | Hypothetical protein |
| **Rv1954A** | 2 | Hypothetical protein |
| **Rv1991A** | 2 | Antitoxin MazE6 |
| **Rv2142A** | 2 | Possible antitoxin ParD2 |
| **Rv2231A** | 2 | Possible toxin VapC16 |
| **Rv2231B** | 2 | Possible antitoxin VapB16 |
| **Rv2274A** | 2 | Possible antitoxin MazE8 |
| **Rv2395A** | 2 | Acid and phagosome regulated protein A AprA |
| **Rv2395B** | 2 | Acid and phagosome regulated protein B AprB |
| **Rv2862A** | 2 | Possible antitoxin VapB23 |
| **Rv3190A** | 2 | Hypothetical protein |
| **Rv3344c** | 2 | PE-PGRS family protein PE_PGRS49] [partial=5'] |
| **Rv3512** | 2 | PE-PGRS family protein PE_PGRS56] [partial=5'] |
| **Rv3599c** | 2 | Hypothetical short protein |

In one aspect, the invention functionally characterizes the potential 'signature sequences (SS)'-carrying candidate genes based on functional information retrieved from Tuberculist (Tuberculosis (Edinb) 91, 7, 2011) and TB database (Nucleic Acids Research, 37, D499, 2009). Accordingly, the potential signature sequences can be functionally characterized into the following groups:
a. 9 (Rv0064A, Rv0456B, Rv0959A, Rv1991A, Rv2142A, **Rv2231A,** Rv2231B, Rv2274A and Rv2862A) fell into the toxin-antitoxin category.
b. 3 (Rv2653c, Rv2654c, Rv2658c) are possible prophages.
c. 2 (Rv3344c and Rv3512) belong to PE_PGRS family of proteins
d. 2 (Rv2645 and Rv2653c) are deleted (partially or completely) in one or more clinical isolates eliminating their use as diagnostic markers.
e. 9 (**Rv1507A**, **Rv1509,** Rv0078B, Rv2645, Rv0397A, Rv1366A, **Rv1954A**, Rv3599c, Rv3190A) are hypothetical proteins.
f. 2 (Rv2395A, Rv2395B) are acid and phagosome regulated proteins.

Based on the *in-silico* analysis, two **Class 1 genes (Rv1507A and Rv1509)** and two **Class 2 genes** (**Rv1954A and Rv2231A)** with homologs in *Mycobacterium bovis BCG* were selected as potential candidates (Table 2).

**Table 2: Nucleotide Sequences of Potential "signature sequences" carrying genes**

| **Gene Name** | **Sequence** | **Description** | **Prediction Class** |
|---|---|---|---|
| **Rv1507A** hypothetical protein (SEQ ID NO: 13) | >Rv1507A | Gene length: 504bp, Protein length: 167aa | **CLASS 1** |
| | ATGCAATCAGGTCAAAATATCCTCGCCAAGGT ATGTAATTTGATTGAACAATCGCGACTTTCTTC AACGCGGTGTCTCCAATTTAGAATAACAAATA CGTCGCGCCCGCGACAGCTCCGCTGGAGCGA GTTCAAGCGATTCTGCGACATATTCAATATGG TGCTCGGGAAGGCCAGGATGGGCCGCGACCC GGGGCGTCCGGTGCGCGATGAACGTCGCATC GTCTCCTGTGAGATAATTGCATCCGATCATAT AGGGCTGGCTGCGGCTAGGTTGCTGGCAAAA AGATATCGCGGCCGATCCGTTTCTGGTTTTGT CTTGATGATCAAATCCGCTTCCGTTCACGAGA TCGATTCCTGGTCTTCCCCCAGCGTCGCGATG TCGATAGGTGTCGCGCTTTGTTCGTACCCGCA CTACGCGGCGGCGAGAACCTCGCCACCGAAT CGGGATTGGGGGGAGGATACCACTCGGTCGA GGCCCGTCACCGGCCTTCTAGCGGGTTG | | |
| **Rv1509**Essen tial hypothetical protein (SEQ ID NO: 14) | >Rv1509 | Gene length: 882bp, Protein length: 293aa | **CLASS 1** |
| | GTGTTTGCGTTGAGTAATAATCTGAACCGTGT GAACGCATGCATGGATGGATTCCTTGCCCGTA TCCGCTCACATGTTGATGCGCACGCGCCAGAA TTGCGTTCACTGTTCGATACGATGGCGGCCGA GGCCCGATTTGCACGCGACTGGCTGTCCGAG GACCTCGCGCGGTTGCCTGTCGGTGCAGCATT | | |
| | GCTGGAAGTGGCGGGGGGTACTTCTGCTC AGCTGTCAACTGGCGGCGGAGGGATTTGACA TCACCGCCATCGAGCCGACGGGTGAAGGTTTT GGCAAGTTCAGACAGCTTGGCGACATCGTGC TGGAATTGGCTGCAGCACGACCCACCATCGC GCCATGCAAGGCGGAAGACTTTATTTCCGAG AAGCGGTTCGACTTCGCCTTCTCGCTGAATGT GATGGAGCACATCGACCTTCCGGATGAGGCA GTCAGGCGGGTATCGGAAGTGCTGAAACCGG GGGCCAGTTACCACTTCCTGTGCCCGAATTAC GTATTCCCGTACGAACCGCATTTCAATATCCCA ACATTCTTCACCAAAGAGCTGACATGCCGGGT GATGCGACATCGCATCGAGGGCAATACGGGC ATGGATGACCCGAAGGGAGTCTGGCGTTCGC TCAACTGGATTACGGTTCCCAAGGTGAAACGC TTTGCGGCGAAGGATGCGACGCTGACCTTGC GCTTCCACCGTGCAATGTTGGTATGGATGCTG GAACGCGCGCTGACGGATAAGGAATTCGCTG GTCGCCGGGCACAATGGATGGTCGCTGCTATT CGCTCGGCGGTGAAATTGCGTGTGCATCATCT GGCAGGCTATGTTCCCGCTACGCTGCAGCCCA TCATGGATGTGCGGCTAACGAAGAGGTAA | | |
| **Rv1954a** Hypothetical protein (SEQ ID NO: 15) | >gi \|448814763:2201231-2201623 Mycobacterium tuberculosis H37Rv complete genome | Gene length: 303bp, Protein length: 100bp | **CLASS 2** |
| | TGGTATAAGCTGGTTTTAGACGAAAAGGACC CCACCTCGGGGTCTGATGGCCAGGGGCAGGG TCGTGTGCATTGGGGATGCAGGTTGCGACTG TACACCCGGCGTGTTCCGCGCGACAGCGGGT | | |
| | GGGATGCCGGTGCTGGTGGTCATCGAGTCTG GGACAGGAGGTGATCAGATGGCTCGTAAAGC TACGTCCCCGGGTAAGCCGGCTCCGACGTCG GGACAGTATCGCCCGGTTGGCGGTGGCAACG AGGTGACCGTTCCGAAGGGACACCGTCTGCC TCCCTCGCCCAAGCCCGGTCAGAAGTGGGTG AACGTCGATCCGACGAAGAACAAGAGCGGCC GCGGCTGAGCTTGTGCCGTCGGGATGGGTGT CGCACCGTCTCGGCGGGTCGC | | |
| **Rv2231A** (SEQ ID NO: 16) | >gi \|448814763:c2506224-2505671 Mycobacterium tuberculosis H37Rv complete genome | Gene length: 426bp, Protein length: 141aa | **CLASS 2** |
| | GCCGCGGCGAGCCGGTAGCAAAGCTTGTGCC GCTGCATCCTCATGAGACTCGGCGGTTAGGCA TTGACCATGGCGTGTACCGCGTGCCCGACGAT TTGGACGCTCCGTTGTCAGACGACGTGCTCGA ACGCTTTCACCGGTGAAGCGCTACCTCATCGA CACCCACGTTTGGCTGCGGATGCCGTCAACGA AACACGGGCGATTGTTCAGGACGTCCGCAAC AGCATTCTCTTGTCGGCCGCCAGTGCCTGGGA GATCGCGATCAACTACCGCCTCGGCAAGCTCC CGCCGCCCGAGCCATCGGCCTCTTACGTGCCC GATCGAATGCGCCGCTGCGGCACGTCGCCGC TGTCAGTTGACCACGCACACACTGCGCACCGC AGAGCTTCCGGATCACCATCGACATCCATTCG ACCGTGTGCTCATCGCCCAGGCACAGCTGCTT GGCCTGACGATCATCACCGCCGACGCCCTGTT AGCTGCCTGTGATGTCGCGGTTGTCGCCGCGT | | |
| | AGACAACGCGTCGGCGGTGCTCTGGATTCTTG GCCCGCACACCG | | |

In a most preferred aspect, the signature sequences were designed keeping in view the diagnostic tool of RT PCR. These were short sequences amenable for PCR amplification from selected genes. The specific signature sequences, SS1 (Rv1507A), SS2 (Rv1509), SS3 (RV1954A) and SS4 (Rv2231A) of the invention are provided below. Homology search using NCBI nucleotide BLAST against the genus Mycobacterium was conducted on these signature sequences to confirm their uniqueness.
**SS1 (Rv1507A): SEQ ID NO: 1**
   **>Rv1507A** 
ATGCAATCAGGTCAAAATATCCTCGCCAAGGTATGTAATTTGATTGAACAATCGCGACTTTCTTCAACGC
GGTGTCTCCAATTTAGAATAACAAATACGTCGCGCCCGCGACAGCTCCGCTGGAGCGAGTTCAAGCGATT
CTGCGACATATTCAATATGGTGCTCGGGAAGGCCAGGATGGGCCGCGACCCGGGGCGTCCGGTGCGCGAT
GAACGTCGCATCGTCTCCTG
**SS2 (Rv1509): SEQ ID NO: 2**
   **>gi |448814763:1700212-1701093 Mycobacterium tuberculosis H37Rv complete genome** 
GTGTTTGCGTTGAGTAATAATCTGAACCGTGTGAACGCATGCATGGATGGATTCCTTGCCCGTATCCGCT
CACATGTTGATGCGCACGCGCCAGAATTGCGTTCACTGTTCGATACGATGGCGGCCGAGGCCCGATTTGC
ACGCGACTGGCTGTCCGAGGACCTCGCGCGGTTGCCTGTCGGTGCAGCATTGCTGGAAGTGGGCGGGGGG
GTACTTCTGCTCAGCTGTCAACTGGCGGCGGAGGGATTTGACATCACCGCCATCGAGCCGACGGGTGAAG
GTTTTGGCAAGTTCAGACAGCTTGGCGACATCGTGCTGGAATTGGCTGCA
**SS3 (RV1954A): SEQ ID NO: 3**
   >gi |448814763:2201277-2201579 Mycobacterium tuberculosis H37Rv complete genome 
ATGGCCAGGGGCAGGGTCGTGTGCATTGGGGATGCAGGTTGCGACTGTACACCCGGCGTGTTCCGCGCGA
CAGCGGGTGGGATGCCGGTGCTGGTGGTCATCGAGTCTGGGACAGGAGGTGATCAGATGGCTCGTAAAGC
TACGTCCCCGGGTAAGCCGGCTCCGACGTCGGGACAGTATCGCCCGGTTGGCGGTGGCAACGAGGTGACC
GTTCCGAAGGGACACCGTCTGCCTCCCTCGCCCAAGCCCGGTCAGAAGTGGGTGAACGTCGATCCGACGA
**SS4 (Rv2231A): SEQ ID NO: 4**
   >gi |448814763:c2506161-2505736 Mycobacterium tuberculosis H37Rv complete genome

TTGACCATGGCGTGTACCGCGTGCCCGACGATTTGGACGCTCCGTTGTCAGACGACGTGCTCGAACGCTT
TCACCGGTGAAGCGCTACCTCATCGACACCCACGTTTGGCTGCGGATGCCGTCAACGAAACACGGGCGAT
TGTTCAGGACGTCCGCAACAGCATTCTCTTGTCGGCCGCCAGTGCCTGGGAGATCGCGATCAACTACCGC
CTCGGCAAGCTCCCGCCGCCCGAGCCATCGGCCTCTTACGTGCCCGATCGAATGCGCCGCTGCGGCACGT
CGCCGCTGTCAGTTGACCACGCACACACTGCGCACCGCAGAGCTTCCGGATCACCATCGACATCCATTCG
ACCGTGTGCTCATCGCCCAGGCACAGCTGCTTGGCCTGA

For the purposes of PCR validation, the signature sequences SS1, SS2, SS3 and SS4 were selected and oligonucleotide primers were designed to generate corresponding specific PCR amplification products. Table 3 provides the set of designed oligonucleotide primers.

**Table 3: Signature sequences SS1, SS2, SS3 and SS4 and respective oligonucleotide primers**

| **Signature Sequences (SS)** | **Sequence** | **Description** | **Prediction Class** |
|---|---|---|---|
| **SS1 from Rv1507A** (SEQ ID NO: 17) | >Rvl507A | NZE_Rvl954A_F | **CLASS 1** |
| | ATGCAATCAGGTCAAAATATCCTCGCC AAGGTATGTAATTTGATTGAACAATCG CGACTTTCTTCAACGCGGTGTCTCCAAT TTAGAATAACAAATACGTCGCGCCCGC GACAGCTCCGCTGGAGCGAGTTCAAGC GATTCTGCGACATATTCAATATGGTGCT CGGGAAGGCCAGGATGGGCCGCGACC CGGGGCGTCCGGTGCGCGATGAACGT CGCATCGTCTCCTG | ATGCAGGTTGCGACTGTA CACCCGG (SEQ ID NO: 5) | |
| | | Length = 25, Tm = 58.6, %G+C = 60 | |
| | | NZE_Rv1954A_R | |
| | | GGCCGCTCTTGTTCTTCGT CGGAT (SEQ ID NO: 6) | |
| | | Length = 24, Tm = 57.4, | |
| | | %G+C = 58.3 | |
| | | Amplicon Size = **^{∼}280 bp** | |
| **SS2 from Rv1509** (SEQ ID NO: 18) | >gi \|448814763:1700212-1701093 | NZE_Rv1509_F | **CLASS 1** |
| | Mycobacterium tuberculosis H37Rv complete genome | GTGTTTGCGTTGAGTAAT AATCTGAACCGTGT (SEQ ID NO: 7) | |
| | GTGTTTGCGTTGAGTAATAATCTGAACC GTGTGAACGCATGCATGGATGGATTCC TTGCCCGTATCCGCTCACATGTTGATGC GCACGCGCCAGAATTGCGTTCACTGTT CGATACGATGGCGGCCGAGGCCCGATT TGCACGCGACTGGCTGTCCGAGGACCT CGCGCGGTTGCCTGTCGGTGCAGCATT GCTGGAAGTGGGCGGGGGGTACTTC TGCTCAGCTGTCAACTGGCGGCGGAGG GATTTGACATCACCGCCATCGAGCCGA CGGGTGAAGGTTTTGGCAAGTTCAGAC AGCTTGGCGACATCGTGCTGGAATTGG CTGCA | | |
| | | Length = 32, Tm = 57.5%G+C = 41 | |
| | | NZE_Rv1509_R | |
| | | AGCCAATTCCAGCACGAT GTCGCC (SEQ ID NO: 8) | |
| | | Length = 24, Tm = 58.8, %G+C = 58.3 | |
| | | Amplicon Size = **^{∼}330bp** | |
| **SS3 from Rv1954A** (SEQ ID NO: 19) | >gi \|448814763:2201277-2201579 Mycobacterium tuberculosis H37Rv complete genome | NZE_Rv1954A_F | **CLASS 2** |
| | ATGGCCAGGGGCAGGGTCGTGTGCATT GGGGATGCAGGTTGCGACTGTACACCC GGCGTGTTCCGCGCGACAGCGGGTGG GATGCCGGTGCTGGTGGTCATCGAGTC TGGGACAGGAGGTGATCAGATGGCTC | ATGCAGGTTGCGACTGTA CACCCGG (SEQ ID NO: 9) | |
| | | Length = 25, Tm = 58.6, %G+C = 60 | |
| | | NZE_Rv1954A_R | |
| | GTAAAGCTACGTCCCCGGGTAAGCCGG CTCCGACGTCGGGACAGTATCGCCCGG TTGGCGGTGGCAACGAGGTGACCGTTC CGAAGGGACACCGTCTGCCTCCCTCGC CCAAGCCCGGTCAGAAGTGGGTGAAC GTCGATCCGACGA | GGCCGCTCTTGTTCTTCGT CGGAT (SEQ ID NO: 10) | |
| | | Length = 24, Tm = 57.4, %G+C = 58.3 | |
| | | Amplicon Size = **^{∼}280 bp** | |
| **SS4 from Rv2231A** (SEQ ID NO: 20) | >gi \|448814763:c2506161-2505736 Mycobacterium tuberculosis H37Rv complete genome | NZE_Rv2231A_F | **CLASS 2** |
| | TTGACCATGGCGTGTACCGCGTGCCCG ACGATTTGGACGCTCCGTTGTCAGACG ACGTGCTCGAACGCTTTCACCGGTGAA GCGCTACCTCATCGACACCCACGTTTGG CTGCGGATGCCGTCAACGAAACACGGG CGATTGTTCAGGACGTCCGCAACAGCA TTCTCTTGTCGGCCGCCAGTGCCTGGG AGATCGCGATCAACTACCGCCTCGGCA AGCTCCCGCCGCCCGAGCCATCGGCCT CTTACGTGCCCGATCGAATGCGCCGCT GCGGCACGTCGCCGCTGTCAGTTGACC ACGCACACACTGCGCACCGCAGAGCTT CCGGATCACCATCGACATCCATTCGACC GTGTGCTCATCGCCCAGGCACAGCTGC TTGGCCTGAC | TGTACCGCGTGCCCGACG ATTTG (SEQ ID NO: 11) | |
| | | Length= 23, Tm = 59.1, %G+C = 61 | |
| | | NZE_Rv2231A_R | |
| | | ACAGGCAGCTAACAGGG CGTCGG (SEQ ID NO: 12) | |
| | | Length = 23, Tm = 57.1, %G+C = 65 | |
| | | Amplicon Size = **^{∼}390 bp** | |

In a preferred embodiment, pathogenic mycobacterium sps can be detected with 100 % specificity following PCR using DNA isolated from clinical samples from patients who presented with clinical symptoms of the disease. In another embodiment, pathogenic mycobacterium sps is also detected using the above method in clinical samples isolated from individuals vaccinated against tuberculosis. In yet another embodiment, pathogenic mycobacterium sps is also detected using the above method in clinical samples isolated from individuals treated against tuberculosis. Pathogenic mycobacterium sps, as provided in the invention, includes *Mycobacterium tuberculosis* and *Mycobacterium bovis.* More specifically, pathogenic mycobacterium sps represents *Mycobacterium tuberculosis,* the TB causing bacterium.
Clinical samples, as meant here, includes specimens such as blood, sputum, cerebrospinal fluid, gastric lavage, tissue biopsies and the likes thereof. PCR product can be easily visualized by any conventional method that can be readily recognized by a person skilled in the art such as electrophoresis.

Following Examples serve as a tool to illustrate the invention. However, it should in no way be considered to be limiting the invention.

### Example 1

### Determination of specificity and sensitivity of signature sequences

### Genomic DNA for PCR amplification

Genomic DNA of *Mycobacterium tuberculosis* and 13 other mycobacterial species were used for testing the specificity of signature sequences using PCR. These include, *M*. *avium* subspecies *paratuberculosis, M. smegmatis* (ATCC19420), *M*. *vaccae, M. marinum* (ATCC927), *M*. *chelonae* (ATCC14472), *M.flavescens* (ATCC14474), *M. fortuitum* (ATCC6481), *M*. *kansasii*(ATCC12478), *M*. *bovis* (ATCC27294), *M. bovis* (BCG), *M*. *avium* (ATCC25291), *M*. *gastri, M. indicuspranii.*

### PCR Reaction

The PCR reaction mixture (50*µl*) consisted of 10xtaqPCR buffer, 0.5mmolMgCI2, 0.4mmol dNTP, 10 pmol forward and reverse primers respectively, 4% DMSO and 1Utaq DNA polymerase.The reaction conditions were the following: 95°C for 5minutes, followed by 35 cycles of 95°C for 30seconds, annealing temperature 50°C for 30seconds, 72° C for 1minute and finally 72°C for 10 minutes. All PCR products were electrophoresed on 2% agarose gel with ethidium bromide staining.

The "signature sequences" were tested for their ability to differentiate *Mycobacterium tuberculosis* DNA from a large number of other mycobacterial DNA in PCR using primers complementary to these "signature sequences" as shown in Table 3. For this purpose, chromosomal DNA extracted from 13 mycobacterium species including human genomic DNA were tested by mycobacteruim genus-specific primers of the 'signature sequences'. SS1 and SS2 were negative for all 13 mycobacterium species tested whereas SS3 and SS4 show positive PCR results only when *M*. *bovis* BCG genomic DNA was used as template.

Table 4 summarizes the specificity data resulting from PCR using specific primers of signature sequences SS1, SS2, SS3 and SS4.

**Table 4: Specific amplification of signature sequences from Mycobacterium tuberculosis and M. bovis BCG**

| **Specimen** | **SS1** | **SS2** | **SS3** | **SS4** |
|---|---|---|---|---|
| *M. tuberculosis* | + | + | + | + |
| *M.bovis BCG* | - | - | + | + |
| *M. avium* | - | - | - | - |
| *M. smegmatis* | - | - | - | - |
| *M. vaccae* | - | - | - | - |
| *M. avium* | - | - | - | - |
| *M. chelonae* | - | - | - | - |
| *M. flori* | - | - | - | - |
| *M. fortuitum* | - | - | - | - |
| *M. kansasi* | - | - | - | - |
| *M. bovis* | - | - | - | - |
| *M. marinum* | - | - | - | - |
| *M. gastri* | - | - | - | - |
| MIP | - | - | - | - |
| MAP | - | - | - | - |
| *M. leprae* | | | | |
| Human Genome | - | - | - | - |

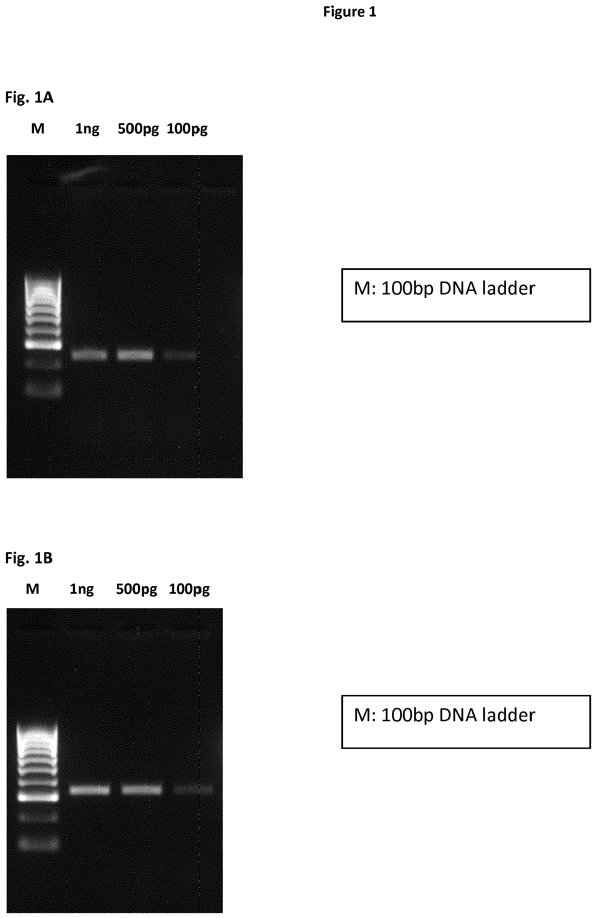

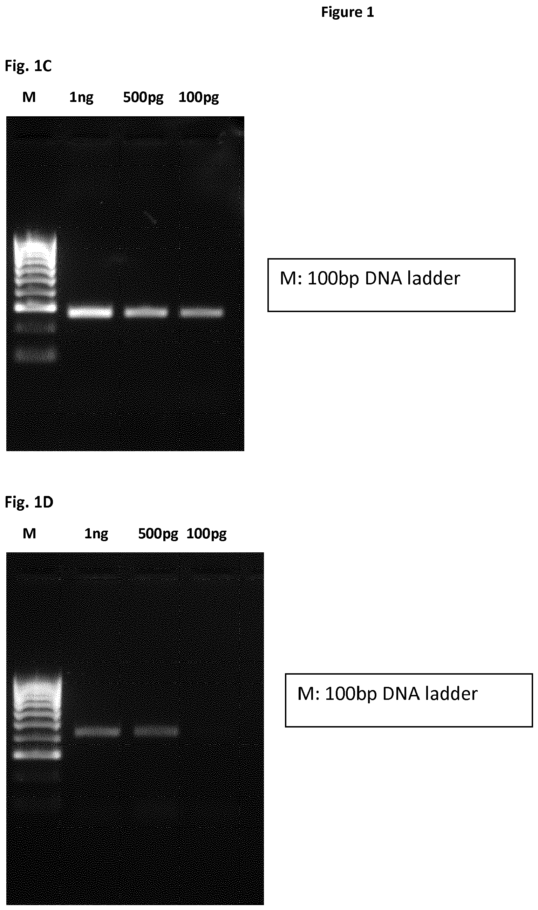

Furthermore, sensitivity analysis revealed that the signature sequences were highly sensitive in being able to detect <1 ng (100pg) DNA as shown in Fig. 1A, 1B, 1C and 1D for the four primers of the signature sequences SS1, SS2, SS3 and SS4 respectively.

### Example 2

### EVALUATION OF MYCOBACTERIUM TUBERCULOSIS-SPECIFIC PRIMER PAIR USING CLINICAL SAMPLES

### A) Amplification of signature sequences from patient sputum samples

Sputum samples were processed by the Universal Sample Processing (USP) method for DNA extraction as described by Chakravorty et al (J Clin Microbiol 43, 4357, 2005). DNA was isolated from the USP sediments by boiling in the presence of five volumes of solution containing 10% Chelex-100 resin, 0.03% triton X-100, and 0.3% Tween 20. The isolated DNA was stored at 20°c and used for PCR assay.

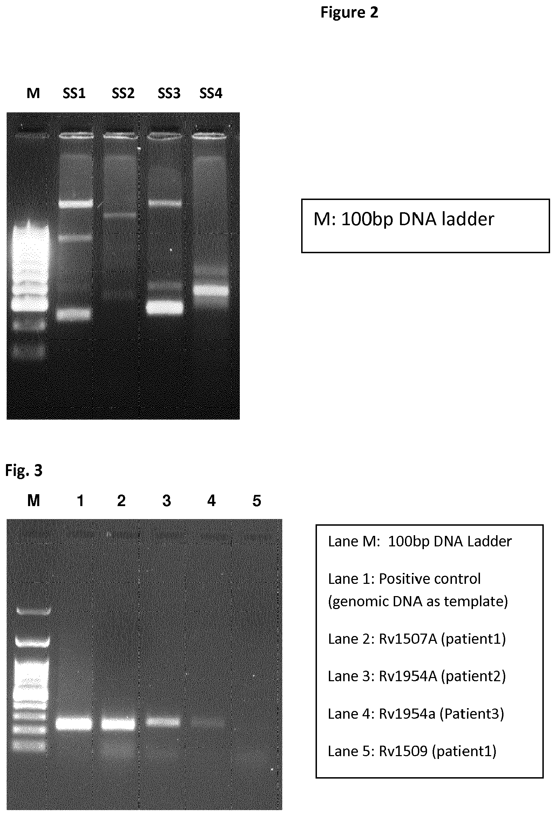

PCR reaction was carried out using specific primers as given in Table 3.
The results show amplification of signature sequences in patient sputum sample (Fig. 2) demonstrating the diagnostic utility of the signature sequences for detecting pathogenic
*Mycobacterium tuberculosis.*

### B) Amplification of signature sequences from patient blood samples

DNA from blood samples of tuberculosis patients were isolated as per the protocol described in van Helden et al (Isolation of DNA from Mycobacterium tuberculosis, Paul D. van Helden, Thomas C. Victor, Robin M. Warren, and Eileen G. van Helden)
The results show amplification of SS1 and SS3 as seen in Fig. 3.

### Sequence Listing

### (1) GENERAL INFORMATION:

### (iii) NUMBER OF SEQUENCES: 4

### (2) INFORMATION FOR SEQ ID NO: 1

### (i) SEQUENCE CHARACTERISTICS:

(A) LENGTH: 230 base pairs
(B) TYPE: Nucleic Acid
(C) STRANDEDNESS: Single
(D) TOPOLOGY: Linear

### (xi) SEQUENCE DESCRIPTION: SEQ ID NO: 1, Rv1507A

1 ATGCAATCAG GTCAAAATAT CCTCGCCAAG GTATGTAATT TGATTGAACA ATCGCGACTT
61 TCTTCAACGC GGTGTCTCCA ATTTAGAATA ACAAATACGT CGCGCCCGCG ACAGCTCCGC
121 TGGAGCGAGT TCAAGCGATT CTGCGACATA TTCAATATGG TGCTCGGGAA GGCCAGGATG
181 GGCCGCGACC CGGGGCGTCC GGTGCGCGAT GAACGTCGCA TCGTCTCCTG

### (2) INFORMATION FOR SEQ ID NO: 2

### (i) SEQUENCE CHARACTERISTICS:

(A) LENGTH: 330 base pairs
(B) TYPE: Nucleic Acid
(C) STRANDEDNESS: Single
(D) TOPOLOGY: Linear

### (xi) SEQUENCE DESCRIPTION: SEQ ID NO: 2, Rv1509

1 GTGTTTGCGT TGAGTAATAA TCTGAACCGT GTGAACGCAT GCATGGATGG ATTCCTTGCC
61 CGTATCCGCT CACATGTTGA TGCGCACGCG CCAGAATTGC GTTCACTGTT CGATACGATG
121 GCGGCCGAGG CCCGATTTGC ACGCGACTGG CTGTCCGAGG ACCTCGCGCG GTTGCCTGTC
181 GGTGCAGCAT TGCTGGAAGT GGGCGGGGGG GTACTTCTGC TCAGCTGTCA ACTGGCGGCG
241 GAGGGATTTG ACATCACCGC CATCGAGCCG ACGGGTGAAG GTTTTGGCAA GTTCAGACAG
301 CTTGGCGACA TCGTGCTGGA ATTGGCTGCA

### (2) INFORMATION FOR SEQ ID NO: 3

### (i) SEQUENCE CHARACTERISTICS:

(A) LENGTH: 280 base pairs
(B) TYPE: Nucleic Acid
(C) STRANDEDNESS: Single
(D) TOPOLOGY: Linear

### (xi) SEQUENCE DESCRIPTION: SEQ ID NO: 3, RV1954A

1 ATGGCCAGGG GCAGGGTCGT GTGCATTGGG GATGCAGGTT GCGACTGTAC ACCCGGCGTG
61 TTCCGCGCGA CAGCGGGTGG GATGCCGGTG CTGGTGGTCA TCGAGTCTGG GACAGGAGGT
121 GATCAGATGG CTCGTAAAGC TACGTCCCCG GGTAAGCCGG CTCCGACGTC GGGACAGTAT
181 CGCCCGGTTG GCGGTGGCAA CGAGGTGACC GTTCCGAAGG GACACCGTCT GCCTCCCTCG
241 CCCAAGCCCG GTCAGAAGTG GGTGAACGTC GATCCGACGA

### (2) INFORMATION FOR SEQ ID NO: 4

### (i) SEQUENCE CHARACTERISTICS:

(A) LENGTH: 389 base pairs
(B) TYPE: Nucleic Acid
(C) STRANDEDNESS: Single
(D) TOPOLOGY: Linear

### (xi) SEQUENCE DESCRIPTION: SEQ ID NO: 4, Rv2231A

1 TTGACCATGG CGTGTACCGC GTGCCCGACG ATTTGGACGC TCCGTTGTCA GACGACGTGC

61 TCGAACGCTT TCACCGGTGA AGCGCTACCT CATCGACACC CACGTTTGGC TGCGGATGCC
121 GTCAACGAAA CACGGGCGAT TGTTCAGGAC GTCCGCAACA GCATTCTCTT GTCGGCCGCC
181 AGTGCCTGGG AGATCGCGAT CAACTACCGC CTCGGCAAGC TCCCGCCGCC CGAGCCATCG
241 GCCTCTTACG TGCCCGATCG AATGCGCCGC TGCGGCACGT CGCCGCTGTC AGTTGACCAC
301 GCACACACTG CGCACCGCAG AGCTTCCGGA TCACCATCGA CATCCATTCG ACCGTGTGCT
361 CATCGCCCAG GCACAGCTGC TTGGCCTGA

SEQUENCE LISTING

<110>  Kusuma School of Biological Sciences
       European Molecular Biology Laboratory

<120>  Genetic markers for diagnosis of tuberculosis caused by
       mycobacterium tuberculosis

<130>  492-61 PCTEPT1

<140>  PCT/IB2014/066469
<141>  2014-12-01

<150>  IN 5572/CHE/2013 3
<151>  2013-12-03

<150>  EP 14 825 438.6
<151>  2014-12-01

<160>  20

<170>  PatentIn version 3.5

<210>  1
<211>  230
<212>  DNA
<213>  Mycobacterium tuberculosis

<400>  1
atgcaatcag gtcaaaatat cctcgccaag gtatgtaatt tgattgaaca atcgcgactt      60

tcttcaacgc ggtgtctcca atttagaata acaaatacgt cgcgcccgcg acagctccgc     120

tggagcgagt tcaagcgatt ctgcgacata ttcaatatgg tgctcgggaa ggccaggatg     180

ggccgcgacc cggggcgtcc ggtgcgcgat gaacgtcgca tcgtctcctg                230


<210>  2
<211>  330
<212>  DNA
<213>  Mycobacterium tuberculosis

<400>  2
gtgtttgcgt tgagtaataa tctgaaccgt gtgaacgcat gcatggatgg attccttgcc      60

cgtatccgct cacatgttga tgcgcacgcg ccagaattgc gttcactgtt cgatacgatg     120

gcggccgagg cccgatttgc acgcgactgg ctgtccgagg acctcgcgcg gttgcctgtc     180

ggtgcagcat tgctggaagt gggcgggggg gtacttctgc tcagctgtca actggcggcg     240

gagggatttg acatcaccgc catcgagccg acgggtgaag gttttggcaa gttcagacag     300

cttggcgaca tcgtgctgga attggctgca                                      330


<210>  3
<211>  280
<212>  DNA
<213>  Mycobacterium tuberculosis

<400>  3

atggccaggg gcagggtcgt gtgcattggg gatgcaggtt gcgactgtac acccggcgtg 60

ttccgcgcga cagcgggtgg gatgccggtg ctggtggtca tcgagtctgg gacaggaggt 120

gatcagatgg ctcgtaaagc tacgtccccg ggtaagccgg ctccgacgtc gggacagtat 180

cgcccggttg gcggtggcaa cgaggtgacc gttccgaagg gacaccgtct gcctccctcg 240

cccaagcccg gtcagaagtg ggtgaacgtc gatccgacga 280

<210> 4
<211> 389
<212> DNA
<213> Mycobacterium tuberculosis

<400> 4
ttgaccatgg cgtgtaccgc gtgcccgacg atttggacgc tccgttgtca gacgacgtgc 60

tcgaacgctt tcaccggtga agcgctacct catcgacacc cacgtttggc tgcggatgcc 120

gtcaacgaaa cacgggcgat tgttcaggac gtccgcaaca gcattctctt gtcggccgcc 180

agtgcctggg agatcgcgat caactaccgc ctcggcaagc tcccgccgcc cgagccatcg 240

gcctcttacg tgcccgatcg aatgcgccgc tgcggcacgt cgccgctgtc agttgaccac 300

gcacacactg cgcaccgcag agcttccgga tcaccatcga catccattcg accgtgtgct 360

catcgcccag gcacagctgc ttggcctga 389

<210> 5
<211> 25
<212> DNA
<213> Mycobacterium tuberculosis

<400> 5
atgcaggttg cgactgtaca cccgg 25

<210> 6
<211> 24
<212> DNA
<213> Mycobacterium tuberculosis

<400> 6
taggctgctt cttgttctcg ccgg 24

<210> 7
<211> 32
<212> DNA
<213> Mycobacterium tuberculosis

<400> 7
gtgtttgcgt tgagtaataa tctgaaccgt gt 32

<210> 8
<211> 24
<212> DNA
<213> Mycobacterium tuberculosis

<400>  8
ccgctgtagc acgaccttaa ccga                                              24


<210>  9
<211>  25
<212>  DNA
<213>  Mycobacterium tuberculosis

<400>  9
atgcaggttg cgactgtaca cccgg                                             25


<210>  10
<211>  24
<212>  DNA
<213>  Mycobacterium tuberculosis

<400>  10
taggctgctt cttgttctcg ccgg                                              24


<210>  11
<211>  23
<212>  DNA
<213>  Mycobacterium tuberculosis

<400>  11
tgtaccgcgt gcccgacgat ttg                                               23


<210>  12
<211>  23
<212>  DNA
<213>  Mycobacterium tuberculosis

<400>  12
ggctgcggga caatcgacgg aca                                               23


<210>  13
<211>  503
<212>  DNA
<213>  Mycobacterium tuberculosis


<220>
<221>  misc_feature
<223>  Rv1507A hypthetical protein

<400>  13
atgcaatcag gtcaaaatat cctcgccaag gtatgtaatt tgattgaaca atcgcgactt      60

tcttcaacgc ggtgtctcca atttagaata acaaatacgt cgcgcccgcg acagctccgc     120

tggagcgagt tcaagcgatt ctgcgacata ttcaatatgg tgctcgggaa ggccaggatg     180

ggccgcgacc cggggcgtcc ggtgcgcgat gaacgtcgca tcgtctcctg tgagataatt     240

gcatccgatc atatagggct ggctgcggct aggttgctgg caaaaagata tcgcggccga     300

tccgtttctg gttttgtctt gatgatcaaa tccgcttccg ttcacgagat cgattcctgg     360

tcttccccca gcgtcgcgat gtcgataggt gtcgcgcttt gttcgtaccc gcactacgcg      420

gcggcgagaa cctcgccacc gaatcgggat tggggggagg ataccactcg gtcgaggccc      480

gtcaccggcc ttctagcggg ttg                                              503

<210>  14
<211>  882
<212>  DNA
<213>  Mycobacterium tuberculosis

<220>
<221>  misc_feature
<223>  Rv1509 Essential hypothetical protein

<400>  14
gtgtttgcgt tgagtaataa tctgaaccgt gtgaacgcat gcatggatgg attccttgcc       60

cgtatccgct cacatgttga tgcgcacgcg ccagaattgc gttcactgtt cgatacgatg      120

gcggccgagg cccgatttgc acgcgactgg ctgtccgagg acctcgcgcg gttgcctgtc      180

ggtgcagcat tgctggaagt gggcgggggg gtacttctgc tcagctgtca actggcggcg      240

gagggatttg acatcaccgc catcgagccg acgggtgaag gttttggcaa gttcagacag      300

cttggcgaca tcgtgctgga attggctgca gcacgaccca ccatcgcgcc atgcaaggcg      360

gaagacttta tttccgagaa gcggttcgac ttcgccttct cgctgaatgt gatggagcac      420

atcgaccttc cggatgaggc agtcaggcgg gtatcggaag tgctgaaacc gggggccagt      480

taccacttcc tgtgcccgaa ttacgtattc ccgtacgaac cgcatttcaa tatcccaaca      540

ttcttcacca aagagctgac atgccgggtg atgcgacatc gcatcgaggg caatacgggc      600

atggatgacc cgaagggagt ctggcgttcg ctcaactgga ttacggttcc caaggtgaaa      660

cgctttgcgg cgaaggatgc gacgctgacc ttgcgcttcc accgtgcaat gttggtatgg      720

atgctggaac gcgcgctgac ggataaggaa ttcgctggtc gccgggcaca atggatggtc      780

gctgctattc gctcggcggt gaaattgcgt gtgcatcatc tggcaggcta tgttcccgct      840

acgctgcagc ccatcatgga tgtgcggcta acgaagaggt aa                         882

<210>  15
<211>  392
<212>  DNA
<213>  Mycobacterium tuberculosis

<220>
<221>  misc_feature
<223>  Rv1954a Hypothetical protein

<400>  15
ggtataagct ggttttagac gaaaaggacc ccacctcggg gtctgatggc caggggcagg       60

gtcgtgtgca ttggggatgc aggttgcgac tgtacacccg gcgtgttccg cgcgacagcg 120

ggtgggatgc cggtgctggt ggtcatcgag tctgggacag gaggtgatca gatggctcgt 180

aaagctacgt ccccgggtaa gccggctccg acgtcgggac agtatcgccc ggttggcggt 240

ggcaacgagg tgaccgttcc gaagggacac cgtctgcctc cctcgcccaa gcccggtcag 300

aagtgggtga acgtcgatcc gacgaagaac aagagcggcc gcggctgagc ttgtgccgtc 360

gggatgggtg tcgcaccgtc tcggcgggtc gc 392

<210> 16
<211> 553
<212> DNA
<213> Mycobacterium tuberculosis

<220>
<221> misc_feature
<223> Rv2231A

<400> 16
gccgcggcga gccggtagca aagcttgtgc cgctgcatcc tcatgagact cggcggttag 60

gcattgacca tggcgtgtac cgcgtgcccg acgatttgga cgctccgttg tcagacgacg 120

tgctcgaacg ctttcaccgg tgaagcgcta cctcatcgac acccacgttt ggctgcggat 180

gccgtcaacg aaacacgggc gattgttcag gacgtccgca acagcattct cttgtcggcc 240

gccagtgcct gggagatcgc gatcaactac cgcctcggca agctcccgcc gcccgagcca 300

tcggcctctt acgtgcccga tcgaatgcgc cgctgcggca cgtcgccgct gtcagttgac 360

cacgcacaca ctgcgcaccg cagagcttcc ggatcaccat cgacatccat tcgaccgtgt 420

gctcatcgcc caggcacagc tgcttggcct gacgatcatc accgccgacg ccctgttagc 480

tgcctgtgat gtcgcggttg tcgccgcgta gacaacgcgt cggcggtgct ctggattctt 540

ggcccgcaca ccg 553

<210> 17
<211> 230
<212> DNA
<213> Mycobacterium tuberculosis

<220>
<221> misc_feature
<223> SS1 from Rv1507A

<400> 17
atgcaatcag gtcaaaatat cctcgccaag gtatgtaatt tgattgaaca atcgcgactt 60

tcttcaacgc ggtgtctcca atttagaata acaaatacgt cgcgcccgcg acagctccgc 120

tggagcgagt tcaagcgatt ctgcgacata ttcaatatgg tgctcgggaa ggccaggatg 180

ggccgcgacc cggggcgtcc ggtgcgcgat gaacgtcgca tcgtctcctg 230

<210>  18
<211>  330
<212>  DNA
<213>  Mycobacterium tuberculosis

<220>
<221>  misc_feature
<223>  SS2 from Rv1509

<400>  18
gtgtttgcgt tgagtaataa tctgaaccgt gtgaacgcat gcatggatgg attccttgcc      60

cgtatccgct cacatgttga tgcgcacgcg ccagaattgc gttcactgtt cgatacgatg     120

gcggccgagg cccgatttgc acgcgactgg ctgtccgagg acctcgcgcg gttgcctgtc     180

ggtgcagcat tgctggaagt gggcgggggg gtacttctgc tcagctgtca actggcggcg     240

gagggatttg acatcaccgc catcgagccg acgggtgaag gttttggcaa gttcagacag     300

cttggcgaca tcgtgctgga attggctgca                                      330

<210>  19
<211>  280
<212>  DNA
<213>  Mycobacterium tuberculosis

<220>
<221>  misc_feature
<223>  SS3 from Rv1954A

<400>  19
atggccaggg gcagggtcgt gtgcattggg gatgcaggtt gcgactgtac acccggcgtg      60

ttccgcgcga cagcgggtgg gatgccggtg ctggtggtca tcgagtctgg gacaggaggt     120

gatcagatgg ctcgtaaagc tacgtccccg ggtaagccgg ctccgacgtc gggacagtat     180

cgcccggttg gcggtggcaa cgaggtgacc gttccgaagg gacaccgtct gcctccctcg     240

cccaagcccg gtcagaagtg ggtgaacgtc gatccgacga                           280

<210>  20
<211>  390
<212>  DNA
<213>  Mycobacterium tuberculosis

<220>
<221>  misc_feature
<223>  SS4 from Rv2231A

<400>  20
ttgaccatgg cgtgtaccgc gtgcccgacg atttggacgc tccgttgtca gacgacgtgc      60

tcgaacgctt tcaccggtga agcgctacct catcgacacc cacgtttggc tgcggatgcc     120

gtcaacgaaa cacgggcgat tgttcaggac gtccgcaaca gcattctctt gtcggccgcc 180

agtgcctggg agatcgcgat caactaccgc ctcggcaagc tcccgccgcc cgagccatcg 240

gcctcttacg tgcccgatcg aatgcgccgc tgcggcacgt cgccgctgtc agttgaccac 300

gcacacactg cgcaccgcag agcttccgga tcaccatcga catccattcg accgtgtgct 360

catcgcccag gcacagctgc ttggcctgac 390

## Claims

1. A nucleotide sequence capable of selectively detecting pathogenic Mycobacterium sps using oligonucleotide primers corresponding to the signature sequence selected from SEQ ID NO: 1, 2, 3 or 4.

2. A method of detecting pathogenic mycobacterium sps in a clinical sample, said method comprising the steps of:
a. removal of contaminants from the clinical sample by conventional methods;
b. extraction of genomic DNA from the contaminant-free clinical sample;
c. designing a set of specific oligonucleotide primers capable of specifically detecting SEQ ID NO: 1, 2, 3 or 4 for use in RT-PCR;
d. analyzing PCR product by electrophoresis or specific probe nucleotide sequence complementary to SEQ ID NO: 1, 2, 3 or 4.

3. The method of claim 2 wherein the set of oligonucleotide primers are selected from
(i) 5' ATGCAGGTTGCGACTGTACACCCGG 3' (SEQ ID NO: 5) 3' GGCCGCTCTTGTTCTTCGTCGGAT 5' (SEQ ID NO: 6)
(ii) 5' GTGTTTGCGTTGAGTAATAATCTGAACCGTGT 3' (SEQ ID NO: 7) 3' AGCCAATTCCAGCACGATGTCGCC 5' (SEQ ID NO: 8)
(iii) 5' ATGCAGGTTGCGACTGTACACCCGG 3' (SEQ ID NO: 9) 3' GGCCGCTCTTGTTCTTCGTCGGAT 5' (SEQ ID NO: 10)
(iv) 5' TGTACCGCGTGCCCGACGATTTG 3' (SEQ ID NO: 11) 3' ACAGGCAGCTAACAGGGCGTCGG 5' (SEQ ID NO: 12)
(v) a set of oligonucleotide primers complementary to (i), (ii), (iii) or (iv)
or
(vi) a set of oligonucleotide primers comprising of sequence containing any 10 consecutive bases from one of the sequences selected from SEQ ID NO: 1, 2, 3 or 4.

4. A kit for detection of pathogenic mycobacterium sps in clinical samples, said kit comprising set of oligonucleotide primers selected from
(i) 5' ATGCAGGTTGCGACTGTACACCCGG 3' (SEQ ID NO: 5) 3' GGCCGCTCTTGTTCTTCGTCGGAT 5' (SEQ ID NO: 6)
(ii) 5' GTGTTTGCGTTGAGTAATAATCTGAACCGTGT 3' (SEQ ID NO: 7) 3' AGCCAATTCCAGCACGATGTCGCC 5' (SEQ ID NO: 8)
(iii) 5' ATGCAGGTTGCGACTGTACACCCGG 3' (SEQ ID NO: 9) 3' GGCCGCTCTTGTTCTTCGTCGGAT 5' (SEQ ID NO: 10)
(iv) 5' TGTACCGCGTGCCCGACGATTTG 3' (SEQ ID NO: 11) 3' ACAGGCAGCTAACAGGGCGTCGG 5' (SEQ ID NO: 12)
(v) a set of oligonucleotide primers complementary to (i), (ii), (iii) or (iv)
or
(vi) a set of oligonucleotide primers comprising of sequence containing any 10 consecutive bases from one of the sequences selected from SEQ ID NO: 1, 2, 3 or 4.

5. A method of detecting pathogenic mycobacterium sps in a clinical sample as in claim 2 wherein the sample is isolated from individuals vaccinated against tuberculosis.

6. A method of detecting pathogenic mycobacterium sps in a clinical sample as in claim 2 wherein the sample is isolated from individuals treated against tuberculosis.
